# EUROPÄISCHE PATENTSCHRIFT

(11) **EP 0 590 458 B1**
(45) Veröffentlichungstag und Bekanntmachung des Hinweises auf die Patenterteilung: **24.02.1999**
(21) Anmeldenummer: 93115087.4
(22) Anmeldetag: 20.09.1993
(51) Int. Cl.: C07D 333/38, A01N 43/10

(54) **Substituierte Thiophencarbonsäureamide**
Substituted thiophene carboxylic acid amides
Amides d'acide thiophène carboxylique substitués

(30) Priorität: 02.10.1992 DE 4233198
(43) Veröffentlichungstag der Anmeldung: 06.04.1994
(73) Patentinhaber: BAYER AG, 51368 Leverkusen (DE)
(72) Erfinder: Elbe, Hans-Ludwig, Dr., D-42329 Wuppertal (DE); Tiemann, Ralf, Dr., D-51375 Leverkusen (DE); Hänssler, Gerd, Dr., D-51381 Leverkusen (DE)

(56) Entgegenhaltungen:
- EP-A- 0 423 523
- EP-A- 0 450 355
- US-A- 3 303 201

## Beschreibung

Die Erfindung betrifft neue substituierte Thiophencarbonsäureamide, ein Verfahren zu ihrer Herstellung und ihre Verwendung in Mitteln zur Bekämpfung phytopathogener Pilze.

Es ist bekannt, daß bestimmte Thiophencarbonsäureamid-Derivate wie beispielsweise die Verbindung 3-Chlor-N-(1-phenylethyl)-benzothiophen-2-carboxamid-S,S-dioxid fungizide Eigenschaften besitzen (vergl. z.B. DE-A 38 32 848).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Weiterhin sind bestimmte substituierte Thiophencarbonsäureamide, wie beispielweise die Verbindungen 4,5-Dibrom-thiophen-2-carbonsäure-N-(4-bromphenyl)-amid (CA-Reg. No. 15686-72-3), 4,5-Dibrom-thiophen-2-carbonsäure-N-(2,3-dichlorphenyl)-amid (CA-Reg. No. 15950-37-5) und 4,5-Dibrom-thiophen-2-carbonsäure-N-[2-(3,4-dimethoxyphenyl)-ethyl]-amid (CA-Reg. No. 98215-50-0) bekannt (vergl. z.B. US-A 3.303.201; GB-A 10 85 974; J. Chem. Soc. Perkin Trans. I, 275-281 [1985]). Über eine fungizide Wirksamkeit dieser Verbindungen gegen phytopathogene Pilze ist aber bisher nichts bekannt.

Im einzelnen geht aus der US-A 3 303 201 hervor, daß zahlreiche Thiophencarbonsäureamide, in denen der Heterocyclus in 4- und 5-Position durch Brom substituiert ist, gegen verschiedene Mikroorganismen, wie Fungi und Bakterien, wirksam sind. Speziell erwähnt wird die Bekämpfung von Staphylococcus aureus, unter anderem auch mit 4,5-Dibrom-thiophen-2-carbonsäure-N-(2,3-dichlor-phenyl)-amid. Ein Einsatz gegen phytopathogene Pilze wird aber nicht expressis verbis genannt.

Es wurden neue substituierte Thiophencarbonsäureamide der Formel 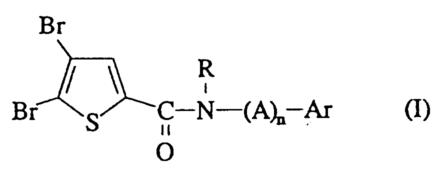 in welcher
- R: für Wasserstoff steht,
- A: für zweifach verknüpftes Methylen steht,
- Ar: für einen Rest der Formel 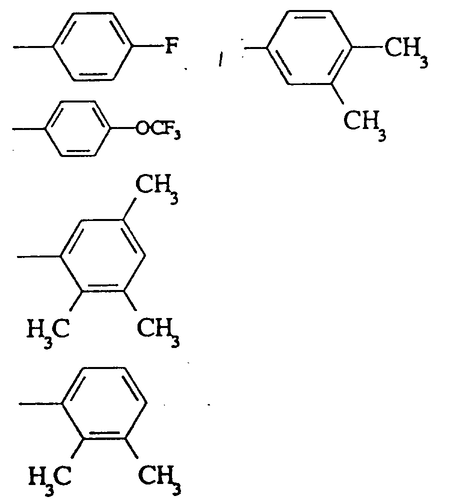 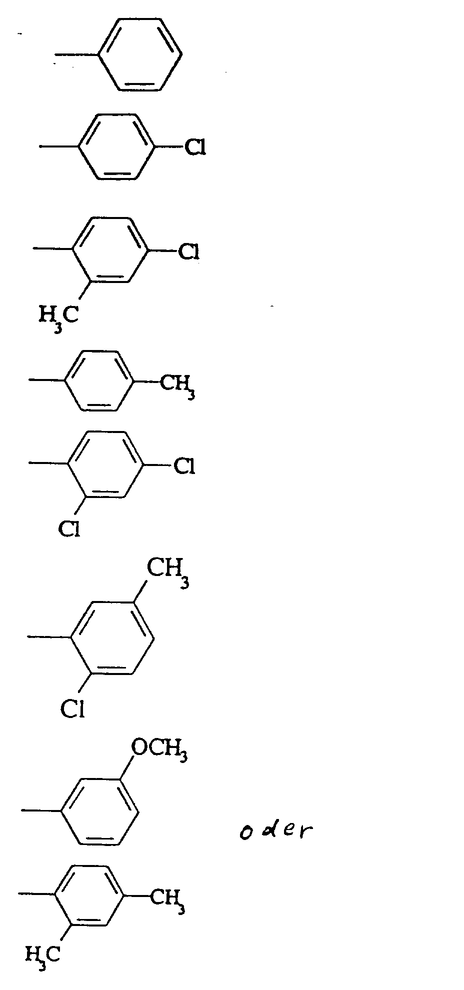 steht und
- n: für eine Zahl 0 oder 1 steht,
gefunden.

Weiterhin wurde gefunden daß man die neuen substituierten Thiophencarbonsäureamide der Formel (I)
erhält wenn man Thiophencarbonsäurehalogenide der Formel 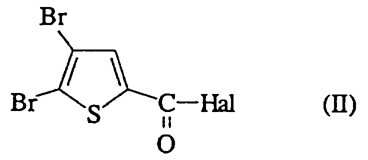 in welcher
- Hal: für Chlor oder Brom steht,
mit Aminen der Formel

R―NH-(A)ₙ―Ar (III)

in welcher
R, A, Ar und n die oben angegebene Bedeutung haben,
gegebenenfalls in Gegenwart eines Verdünnungsmittels und gegebenenfalls in Gegenwart eines Säurebindemittels umsetzt.

Schließlich wurde gefunden, daß die neuen substituierten Thiophencarbonsäureamide der Formel (I) gute Wirksamkeit gegen phytopathogene Pilze besitzen.

Überraschenderweise zeigen die erfindungsgemäßen substituierten Thiophencarbonsäureamide der Formel (I) eine erheblich bessere Wirksamkeit gegenüber phytopathogenen Pilzen im Vergleich zu den aus dem Stand der Technik bekannten Thiophencarbonsäureamid-Derivaten, wie beispielsweise die Verbindung 3-Chlor-N-(1-phenylethyl)-benzothiophen-2-carboxamid-S,S-dioxid, welches chemisch und/oder wirkungsmäßig naheliegende Verbindungen sind.

Verwendet man beispielsweise 4,5-Dibrom-thiophen-2-carbonsäurechlorid und 3,4-Dimethylanilin als Ausgangsverbindungen, so läßt sich der Reaktionsablauf des erfindungsgemäßen Verfahrens durch das folgende Formelschema darstellen:

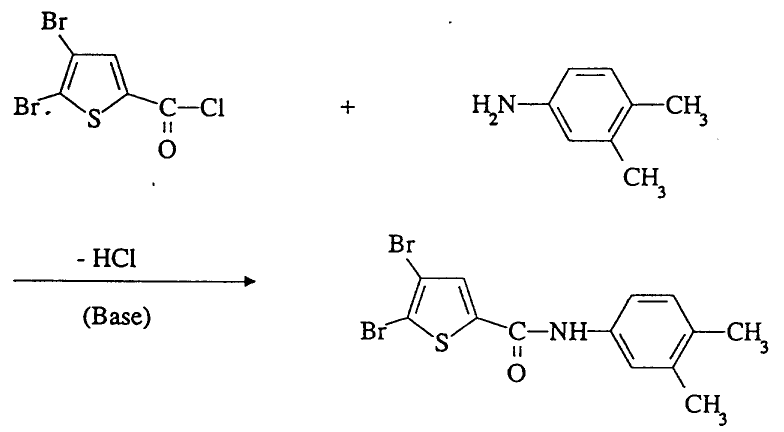

Die zur Durchführung des erfindungsgemäßen Verfahrens als Ausgangsstoffe benötigten Thiophencarbonsäurehalogenide der Formel (II) sind bekannt (vergl. z.B. EP-A 450 355; US-A 3.303.201; DE-A 12 01 952; GB-A 10 85 974).

Die zur Durchführung des erfindungsgemäßen Verfahrens weiterhin als Ausgangsstoffe benötigten Amine der Formel (III) sind allgemein bekannte Verbindungen der organischen Chemie oder erhältlich in Analogie zu allgemein bekannten Verfahren.

Als Verdünnungsmittel zur Durchführung des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff, Ether, wie Diethylether, Diisopropylether, Dioxan, Tetrahydrofuran oder Ethylenglykoldimethyl oder -diethylether; Ketone, wie Aceton, Butanon oder Methylisobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester oder Sulfoxide, wie Dimethylsulfoxid.

Das erfindungsgemäße Verfahren wird vorzugsweise in Gegenwart eines geeigneten Säurebindemittels durchgeführt. Als solche kommen alle üblichen anorganischen oder organischen Basen infrage. Hierzu gehören beispielsweise Erdalkali- oder Alkalimetallhydride, -amide, -alkoholate, Erdalkalimetall-, Alkalimetall- oder Ammoniumhydroxide, -acetate, -carbonate oder -hydrogencarbonate, wie beispielsweise Natriumhydrid, Nathumsmid, Natriummethylat, Natriumethylat, Kalium-tert,-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin N-Methylpiperidin, N,N-Dimethylaminopyridin, Diazabicyclooctan (DABCO), Diazabicyclononen (DBN) oder Diazabicycloundecen (DBU).

Das erfindungsgemäße Verfahren kann gegebenenfalls auch in einem Zweiphasensystem, wie beispielsweise Wasser/Toluol oder Wasser/Dichlormethan, gegebenenfalls in Gegenwart eines geeigneten Phasentransferkatalysators` durchgeführt werden. Als Beispiele für solche Katalysatoren seien genannt: Tetrabutylammoniumiodid, Tetrabutylammoniumbromid, Tetrabutylammoniumchlorid, Tributyl-methylphosphoniumbromid, Trimethyl-C₁₃/C₁₅-alkylammoniumchlorid, Trimethyl-C₁₃/C₁₅-alkylammoniumbromid, Dibenzyl-dimethyl-ammoniummethylsulfat, Dimethyl-C₁₂/C₁₄-alkyl-benzylammoniumchlorid, Dimethyl-C₁₂/C₁₄-alkyl-benzylammoniumbromid, Tetrabutylammoniumhydroxid, Triethylbenzylammoniumchlorid, Methyltrioctylammoniumchlorid, Trimethylbenzylammoniumchlorid, 15-Krone-5, 18-Krone-6 oder Tris-[2-(2-methoxyethoxy)-ethyl]-amin

Die Reaktionstemperaturen können bei der Durchführung des erfindungsgemäßen Verfahrens in einem größeren Bereich variiert werden. Im allgemeinen arbeitet man bei Temperaturen zwischen -20°C und 150°C, vorzugsweise bei Temperaturen zwischen 0°C und 120°C.

Das erfindungsgemäße Verfahren wird üblicherweise unter Normaldruck durchgeführt Es ist jedoch auch möglich unter erhöhtem oder vermindertem Druck zu arbeiten.

Zur Durchführung des erfindungsgemäßen Verfahrens setzt man pro Mol an Thiophencarbonsäurehalogenid der Formel (II) im allgemeinen 1,0 bis 5,0 Mol, vorzugsweise 1,0 bis 2,5 Mol an Amin der Formel (III) und gegebenenfalls 1,0 bis 5,0 Mol, vorzugsweise 1,0 bis 2,5 Mol an Säurebindemittel ein. Die Reaktionsdurchführung, Aufarbeitung und Isolierung der Reaktionsprodukte erfolgt nach allgemein bekannten Verfahren (vergl. hierzu auch die Herstellungsbeispiele).

Die Reinigung der Endprodukte der Formel (I) erfolgt mit Hilfe üblicher Verfahren, beispielsweise durch Säulenchromatographie oder durch Umkristallisieren.

Die Charakterisierung erfolgt mit Hilfe des Schmelzpunktes oder bei nicht kristallisierenden Verbindungen mit Hilfe des Brechungsindex oder der Protonen-Kernresonanzspektroskopie (¹H-NMR).

Die erfindungsgemäßen Wirkstoffe weisen eine starke Wirkung gegen phytopathogene Pilze auf und können zur Bekämpfung von diesen unerwünschten Schadorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Krankheiten, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder Peronospora brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielweise Pyrenophora teres oder Pyrenophora graminea (Konidienform: Drechslera, Synonym: Helminthosporium);
Cochliobolus-Arten, wie beispielsweise Cochliobolus sativus (Konidienform:
Drechslera, Synonym: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botryis-Arten, wie beispielsweise Botrytis cinerea:
Septoria-Arten, wie beispielsweise Septoria nodorum;
Leptosphaeria-Arten, wie beispielsweise Leptosphaeria nodorum;
Cercospora-Arten, wie beispielsweise Cercospora canescers;
Alternaria-Arten, wie beispielsweise Alternaria brassicae;
Pseudocercosporella-Arten, wie beispielsweise Pseudocercosporella herpothchoides.

Die gute Pflanzenvertäglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Dabei können die erfindungsgemäßen Wirkstoffe mit besonders gutem Erfolg zur Bekämpfung von Krankheiten im Obst- und Gemüseanbau, wie beispielsweise gegen den Erreger des echten Apfelmehltaus (Podosphaera leucotricha) oder zur Bekämpfung von Reiskrakheiten, wie beispielsweise gegen den Erreger der Reisfleckenkrankheit (Pyricularla oryzae) eingesetzt werden. Daneben besitzen die erfindungsgemäßen Wirkstoffe auch eine resistenz-induzierende Wirksamkeit

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und -Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Malskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carböxymethylcellulose, natürliche und synthetische; pulverige, kömige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Falbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinenzwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50g je Kilogramm Saatgut, vorzugsweise 0,01 bis 10 g benötigt.

Bei der Behandlung des Bodens sind Wirkstoftkonzentrationen von 0,00001 bis 0,1 Gew.-%, vorzugsweise von 0,0001 bis 0,02 Gew.-% am Wirkungsort erforderlich.

### Herstellungsbeispiele:

### Beispiel 1:

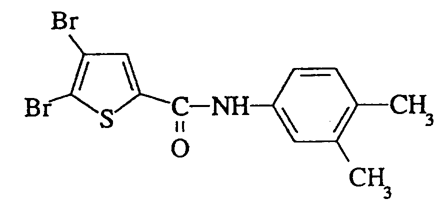

Zu 2,0 g (0,02 Mol) Triethylamin und 2,4 g (0,02 Mol) 3,4-Dimethylanilin in 30 ml Toluol gibt man bei Raumtemperatur unter Rühren eine Lösung von 6,0 g (0,02 Mol) 4,5-Dibromthiophen-2-carbonsäurechlorid zu, erwärmt anschließend für 2 Stunden auf 50°C. Zur Aufarbeitung kühlt man den Reaktionsansatz auf Raumtemperatur ab, gibt die Mischung in Wasser, saugt ausgefallenen Feststoff ab, wäscht nach mit Wasser und n-Hexan und trocknet den Rückstand im Vakuum bei 50°C.

Man erhält 6,4 g (82% der Theorie) an 4,5-Dibromthiophen-2-carbonsäure-N-(3,4-dimethylphenyl)-amid vom Schmelzpunkt 216-217°C.

In entsprechender Weise und gemäß den allgemeinen Angaben zur Herstellung erhält man die folgenden substituierten Thiophencarbonsäureamide der allgemeinen Formel (I):

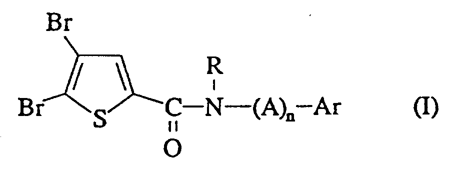

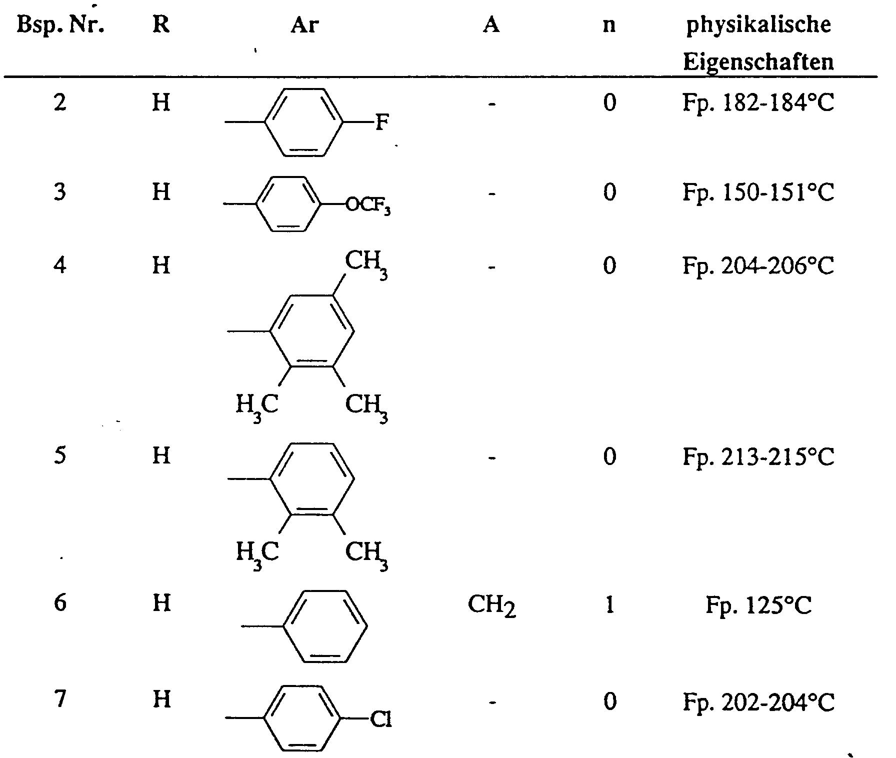

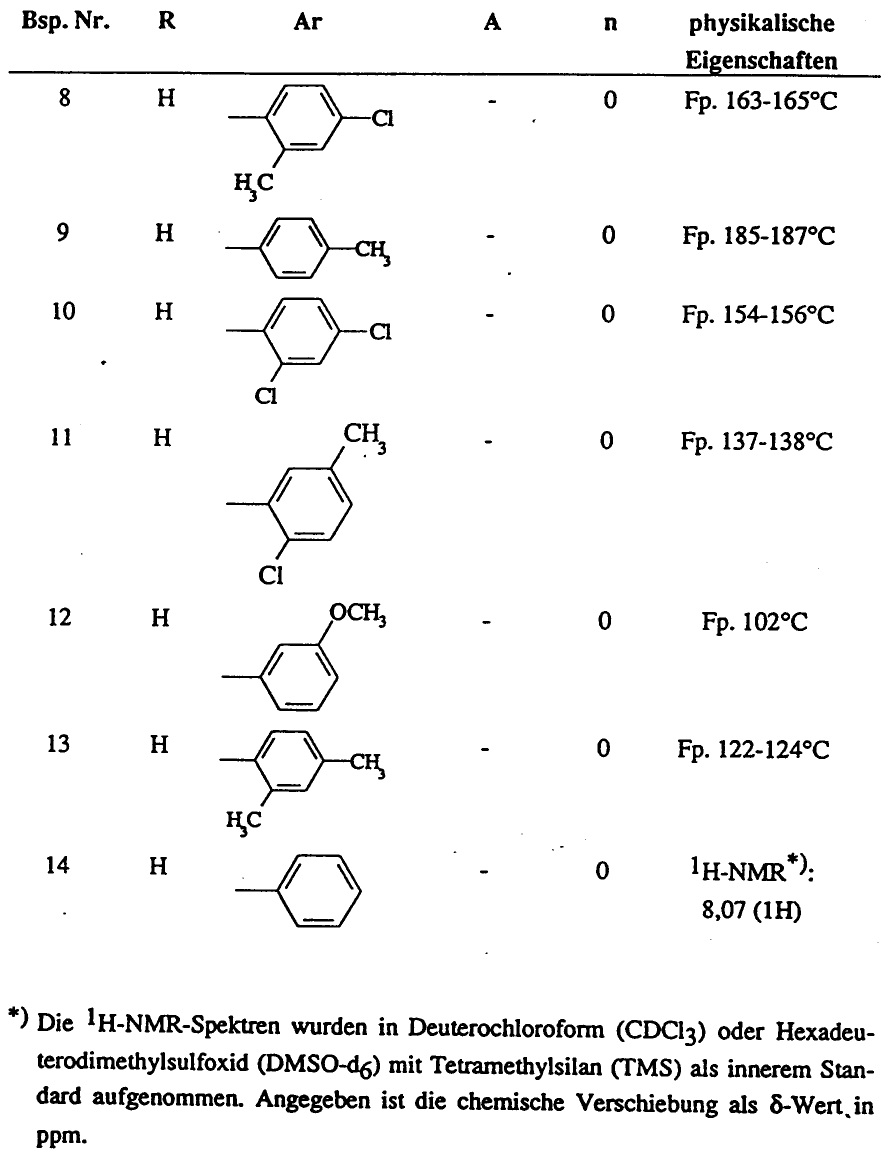

### Anwendungsbeispiele:

In den folgenden Anwendungsbeispielen wurde die nachstehend aufgeführte Verbindung als Vergleichssubstanz eingesetzt: 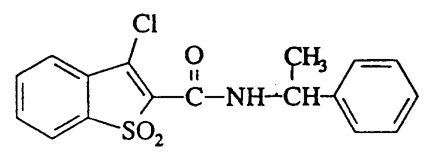 3-Chlor-N-(1-phenylethyl)-henzothiophen-2-carboxamid-S,S-dioxid (bekannt aus DE- A 38 32 848)

### Beispiel A:

### Pyricularia Test (Reis) / protektiv

- Lösungsmittel:: 12,5 Gewichtsteile Aceton
- Emulgator:: 0,3 Gewichtsteile Alkyl-aryl-polyglykolether

Zur Herstellung einer zweckmäßigen Wirkstoffzubereitung vermischt man ein Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach Antrocknen des Spritsbelages werden die Pflanzen mit einer wässrigen Sporensuspension von Pyricularia oryzae inokuliert.

Die Pflanzen werden anschließend im Gewächshaus bei 25°C und einer relativen Luftfeuchtigkeit von 100% aufgestellt

4 Tage nach der Inokulation erfolgt die Auswertung des Krankheitsbefalls.

Bei einer Wirkstoffkonzentration von 0,025 % zeigen die Verbindungen gemäß den Herstellungsbeispielen 6, 13 und 14 einen Wirkungsgrad von mindestens 90% gegenüber der unbehandelten Kontrolle, während die Vergleichssubtanz einen Wirkungsgrad von nur 10% zeigt.

## Patentansprüche

1. Substituierte Thiophencarbonsäureamide der Formel 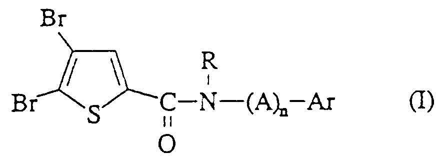 in welcher
R für Wasserstoff steht,
A für zweifach verknüpftes Methylen steht,
Ar für einen Rest der Formel 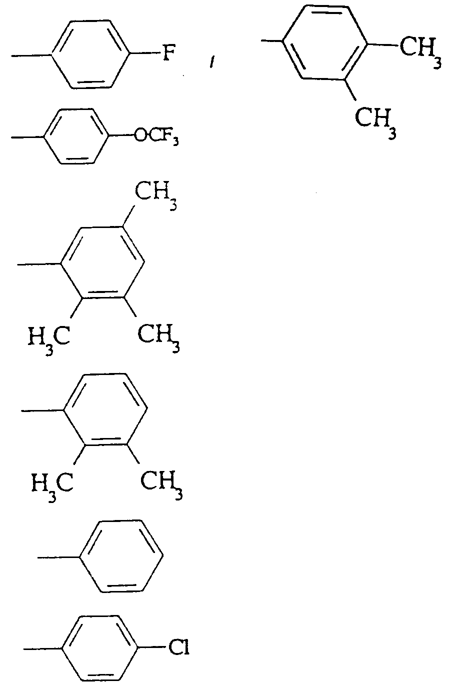 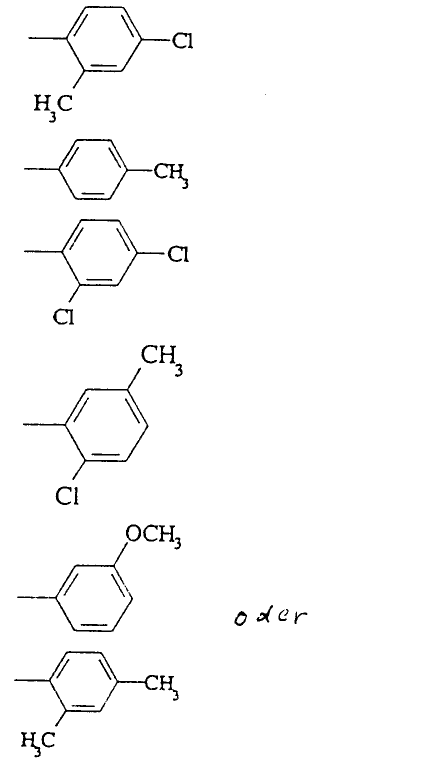 steht und
n für eine Zahl 0 oder 1 steht.

2. Verfahren Zur Herstellung von substituierten Thiophencarbonsäureamiden der Formel (I) gemäß Anspruch 1, dadurch gekennzeichnet, daß man Thiophencarbonsäurehalogenide der Formel 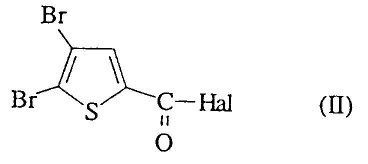 in welcher
Hal für Chlor oder Brom steht,
mit Aminen der Formel
R―NH-(A)ₙ―Ar (III)
in welcher
R, A, Ar und n die oben angegebene Bedeutung haben,
gegebenenfalls in Gegenwart eines Verdünnungsmittels und gegebenenfalls in Gegenwart eines Säurebindemittels umsetzt.

3. Mittel zur Bekämpfung phytopathogener Pilze, gekennzeichnet durch einen Gehalt an mindestens einem substituierten Thiophencarbonsäureamid der Formel (I) gemäß Anspruch 1.

4. Verwendung von substituierten Thiophencarbonsäureamiden der Formel (I) gemäß Anspruch 1 zur Bekämpfung von phytopathogenen Pilzen.

5. Verfahren zur Bekämpfung phytopathogener Pilze, dadurch gekennzeichnet, daß man substituierte Thiophencarbonsäureamide der Formel (I) gemäß Anspruch 1 auf die Pilze und/ oder deren Lebensraum ausbringt.

## Claims

1. Substituted thiophenecarboxamides of the formula 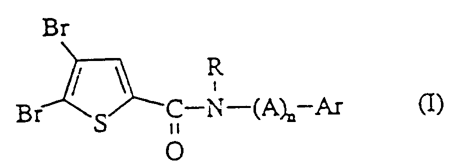 in which
R represents hydrogen,
A represents doubly linked methylene,
Ar represents a radical of the formula 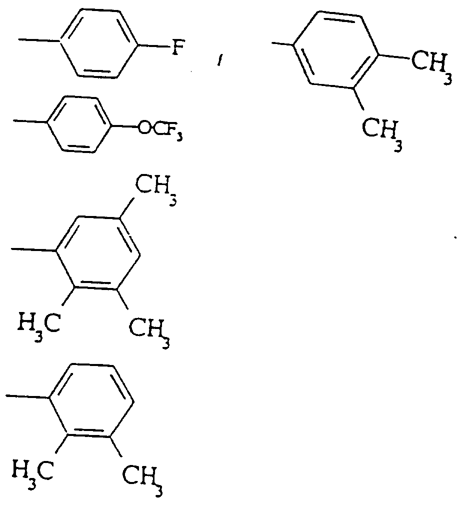 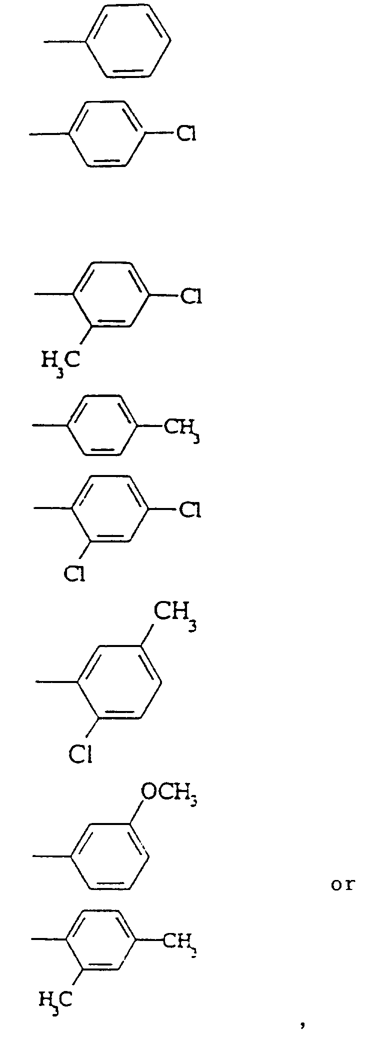 and
n represents a number 0 or 1.

2. Process for preparing substituted thiophenecarboxamides of the formula (I) according to Claim 1, characterized in that thiophenecarbonyl halides of the formula 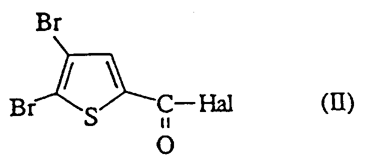 in which
Hal represents chlorine or bromine,
are reacted with amines of the formula
R―NH-(A)ₙ―Ar (III)
in which
R, A, Ar and n have the abovementioned meaning, optionally in the presence of a diluent and optionally in the presence of an acid-binding agent.

3. Composition for controlling phytopathogenic fungi, characterized by a content of at least one substituted thiophenecarboxamide of the formula (I) according to Claim 1.

4. Use of substituted thiophenecarboxamides of the formula (I) according to Claim 1 for controlling phytopathogenic fungi.

5. Process for controlling phytopathogenic fungi, characterized in that substituted thiophenecarboxamides of the formula (I) according to Claim 1 are applied to the fungi and/or their habitat.

## Revendications

1. Amides d'acide thiophène-carboxylique substitués de formule 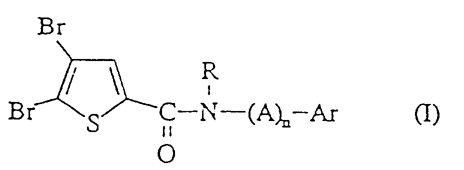 dans laquelle
R représente de l'hydrogène,
A est un groupe méthylène à deux liaisons,
Ar représente un reste de formule 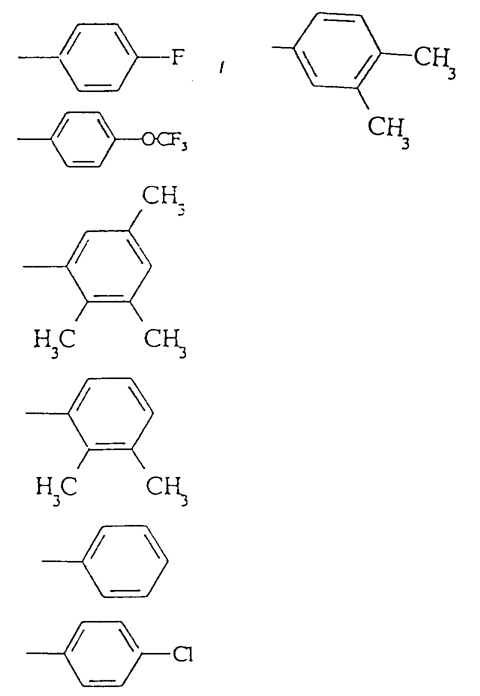 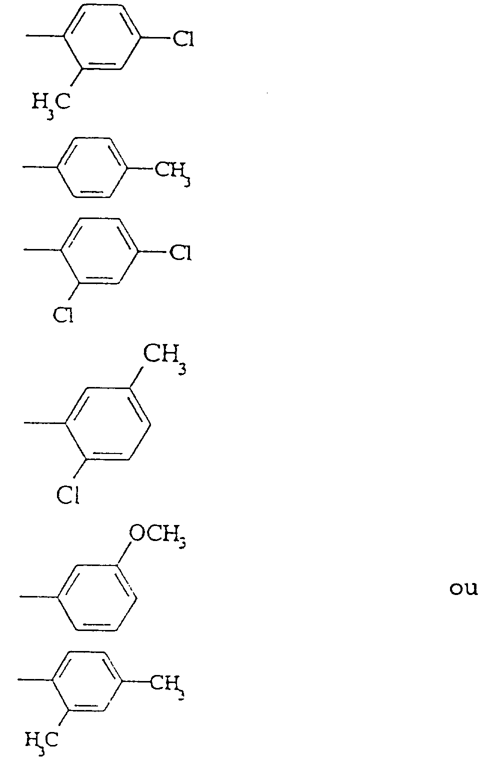 et
n représente le nombre 0 ou 1.

2. Procédé de production d'amides d'acide thiophène-carboxylique substitués de formule (I) suivant la revendication 1
caractérisé en ce qu'on fait réagir des halogénures d'acide thiophène-carboxylique de formule 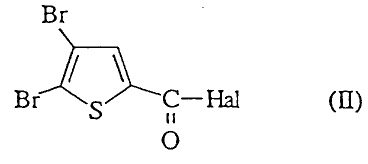 dans laquelle
Hal représente du chlore ou du brome,
avec des amines de formule
R-NH-(A)ₙ-Ar (III)
dans laquelle
R, A, Ar et n ont la définition indiquée ci-dessus,
le cas échéant en présence d'un diluant et en la présence éventuelle d'un accepteur d'acide.

3. Compositions destinées à combattre des champignons phytopathogènes, caractérisées par une teneur en au moins un amide d'acide thiophène-carboxylique substitué de formule (I) suivant la revendication 1.

4. Utilisation d'amides d'acide thiophènecarboxylique substitués de formule (I) suivant la revendication 1 pour combattre des champignons phytopathogènes.

5. Procédé pour combattre des champignons phytopathogènes, caractérisé en ce qu'on applique des amides d'acide thiophènecarboxylique substitués de formule (I) suivant la revendication 1 aux champignons et/ou à leur milieu.
